# EUROPEAN PATENT SPECIFICATION

(11) **EP 2 435 001 B1**
(45) Date of publication and mention of the grant of the patent: **11.09.2013**
(21) Application number: 10714099.8
(22) Date of filing: 09.03.2010
(51) Int. Cl.: A61F 13/15

(54) **PANTS-TYPE DISPOSABLE DIAPER**
EINWEG-WINDEL VOM HÖSCHENTYP
COUCHE JETABLE DE TYPE CULOTTE

(30) Priority: 29.05.2009 JP 2009130886
(43) Date of publication of application: 04.04.2012
(73) Proprietor: Livedo Corporation, Shikokuchuo-shi Ehime 799-0122 (JP)
(72) Inventor: TAKAHASHI, Yuki, Mima-gun Tokushima 779-4104 (JP); NAKAOKA, Kenji, Osaka-shi Osaka 541-0048 (JP)
(74) Representative: Gulde Hengelhaupt Ziebig & Schneider
(86) International application number: PCT/JP2010/054278
(87) International publication number: WO 2010/137382

(56) References cited:
- WO-A1-98/20822
- WO-A1-2010/017158
- JP-A- 2007 097 979
- US-A1- 2003 106 560
- US-A1- 2004 091 677
- US-A1- 2006 135 923

## Description

### TECHNICAL FIELD

The present invention relates to a pants-type disposable diaper for an infant or an adult.

### BACKGROUND ART

Conventinally, there is known a pants-type disposable diaper comprising: a pants-shaped outer member including an inner sheet and an outer sheet; and an absorbent main body disposed on an inner surface of the pants-shaped outer member. Japanese Laid-Open Patent Publication No. 2007-097979 (Patent Literature 1) discloses that a hydrophilized sheet that is prepared by immersing a spunbonded nonwoven fabric formed from a polypropylene or a polyolefin/polyester copolymer in a surfactant, is used in the pants-type disposable diaper as the inner sheet and the outer sheet of the pants-shaped outer member. In addition, Patent Literature 1 discloses that when the spunbonded nonwoven fabric, that is formed from the polypropylene or the polyolefin/polyester copolymer, is immersed in the surfactant, a surface of the nonwoven fabric sheet is smoothed and a skin feel of the nonwoven fabric sheet is improved.

### CITATION LIST

### PATENT LITERATURE

PATENT LITERATURE 1
   Japanese Laid-Open Patent Publication No. 2007-097979

### SUMMARY OF INVENTION

### TECHNICAL PROBLEM

In the pants-type disposable diaper disclosed in Patent Literature 1, a skin feel of the nonwoven fabric, which is used as the inner sheet and the outer sheet of the pants-shaped outer member, is improved by immersing the nonwoven fabric in the surfactant. However, since the nonwoven fabric is hydrophilized at the same time, water resistance of the pants-shaped outer member decreases. Therefore, there is room for improvements in leakage prevention.

WO 2010/017158 A1 which is prior art according to Article 54 (3) discloses an absorbent article that comprises a barrier component of a nonwoven barrier sheet usable for a back sheet, comprising a spunbond nonwoven web and a meltblown nonwoven web. The document further describes that the surface of the barrier component may be coated with a skin care composition, and the skin care composition may comprise a solid nonionic surfactant. Thus, this nonionic surfactant is not melted with a polymer to form a nonwoven web, but is a component of the skin care composition. US 2004/091677 A1 discloses a multimicrolayer thermoplastic film having a plurality of corrugated polymer layers and a plurality of thermoplastic elastomer layers which may comprise filler materials and surfactants. US 2003/0106560 A1 describes a single-use, disposable absorbent laminate containing one or more layers of hydrophilic meltspun material bonded to a breathable film. The meltspun fabric layer may include at least one spunbonded fabric that is made hydrophilic prior to bonding to the film. The filaments or microfibers of the spunbonded or meltblown fabrics may contain a hydrophilic additive in or on the flaments or microfibers. US 2006/0135923 A1 discloses a nonwoven fabric composite that comprises at least one extensible layer of elastomeric fine fibers treated with a wetting agent. The nonwoven fabric composite can be used as barrier layer or a wrap for an absorbent material to prevent particles form migrating out of the absorbent material.

WO 98/20822 A1 describes a disposable absorbent article such as an absorbent diaper comprising a topsheet, a backsheet and an absorbent core disposed between the topsheet and the backsheet. The backsheet comprises nonwoven web. The absorbent article has a softclothlike backsheet which comprises a plastic film having an outer-facing surface and a bodyfacing surface, and the nonwoven web is joined with the outer-facing surface of the plastic film to form a laminate. The disclosed absorbent article does not have an outer member of pants shape.

The present invention has been achieved in view of the above circumstances, and an object of the present invention is to provide a pants-type disposable diaper comprising a pants-shaped outer member that is excellent in water resistance and softness.

### SOLUTION TO PROBLEM

A pant-type disposable diaper of the present invention which solves the above problems comprises: a pants-shaped outer member having a front part, a back part, and a crotch part positioned between the front part and the back part, and having a waist opening and a pair of leg openings formed by joining the front part and the back part; and an absorbent main body disposed on an inner surface of the pants-shaped outer member at the crotch part, and comprising a top sheet, a back sheet, and an absorbent core disposed between the top sheet and the back sheet; wherein the pants-shaped outer member comprises a first sheet made of a spunbonded nonwoven fabric produced by melting a polymer composition containing polyolefin and/or polyester and a nonionic surfactant, a second sheet laminated on an inner surface or an outer surface of the first sheet, made of nonwoven fabric, and not containing a surfactant; and an elastic member disposed between the first sheet and the second sheet. In the present invention, a nonwoven fabric used for the pants-shaped outer member is not obtained by immersing the nonwoven fabric in a surfactant, but is obtained by forming the nonwoven fabric from the polymer composition, a raw material, in which a surfactant is contained. Therefore, hydrophobicity provided by polyolefin and/or polyester is maintained in the obtained nonwoven fabric, and hence the nonwoven fabric is excellent in water resistance, and further softness is imparted to the nonwoven fabric.

The surfactant contained in the polymer composition is a nonionic surfactant. When a nonionic surfactant is used as the surfactant, hydrophobicity of the polyolefin and/or polyester nonwoven fabric tends to be maintained, and softness is easily imparted to the nonwoven fabric.

The nonwoven fabric of the first sheet preferably has a fineness of 1.0 dtex or more and less than 1.5 dtex. When the nonwoven fabric has a fineness of less than 1.5 dtex, softness is easily imparted to the nonwoven fabric. When the nonwoven fabric has a fineness of 1.0 dtex or more, the spunbonded nonwoven fabric is easily produced.

The pants-shaped outer member comprises a second sheet laminated on an inner surface or an outer surface of the first sheet and not containing a surfactant. When the pants-shaped outer member has only the first sheet as a sheet member, the pants-shaped outer member may become excessively soft, thereby deteriorating handleability during manufacturing or wearing of the diaper. For that reason, in light of imparting rigidity to the pants-shaped outer member to improve handleability of the diaper, the pants-shaped outer member has the second sheet in addition to the first sheet.

In the pants-shaped outer member, preferably, the second sheet is laminated on the inner surface of the first sheet, and the first sheet is folded back toward the second sheet at an edge of the waist opening of the pants-shaped outer member. When the second sheet is laminated on the inner surface of the first sheet, the absorbent main body comes to be fixed to the second sheet having a certain degree of rigidity. Therefore, the absorbent main body is easily set at a desired position in a crotch of a wearer in wearing the diaper, thereby improving handleability of the diaper. In addition, when the first sheet is folded back toward the second sheet at the edge of the waist opening, a wearer's skin comes to contact the first sheet at around the waist opening of the pants-shaped outer member, thereby a skin feel of the pants-shaped outer member being improved.

Preferably, a plurality of body elastic members are disposed between the first sheet and the second sheet at the front part and the back part of the pants-shaped outer member so as to extend in a width direction of the diaper, the body elastic members are adhered to the first sheet and/or the second sheet, and the first sheet and the second sheet are not adhered each other at a position between the adjacent body elastic members. The body elastic member improves a fitting property of the diaper around abdomen and hip regions. In addition, when the first sheet and the second sheet are not adhered each other at the position between the adjacent body elastic members, a hand feel of the diaper is further improved around a wearer's trunk.

### 30 ADVANTAGEOUS EFFECTS OF INVENTION

The pants-type disposable diaper of the present invention comprises the pants-shaped outer member that is excellent in water resistance and softness, and hence, leakage of urine and the like is easily prevented, and a hand feel in handling the diaper and a skin feel of the diaper against the wearer are improved.

### BRIEF DESCRIPTION OF DRAWINGS

Fig. 1 shows a perspective view of a pants-type disposable diaper of the present invention.
Fig. 2 shows a plan view of the pants-type disposable diaper shown in Fig. 1 in a developed state in which a front part and a back part are disjoined.
Fig. 3 shows a cross-sectional view taken along line III-III in Fig. 2.
Fig. 4 shows a cross-sectional view taken along line IV-IV in Fig. 2.
Fig. 5 shows a cross-sectional view taken along line V-V in Fig. 2.
Figs. 6A, 6B and 6C show examples of applying a hot-melt adhesive to a body elastic member.

### DESCRIPTION OF EMBODIMENTS

A pants-type disposable diaper of the present invention comprises: a pants-shaped outer member having a front part, a back part, and a crotch part positioned between the front part and the back part, and having a waist opening and a pair of leg openings formed by joining the front part and the back part; and an absorbent main body disposed on an inner surface of the pants-shaped outer member at the crotch part, and comprising a top sheet, a back sheet, and an absorbent core disposed between the top sheet and the back sheet.

In the pants-shaped outer member, the front part and the back part are joined at their both side edges in a width direction of the diaper, whereby forming the pair of leg openings on both sides of the crotch part and the waist opening provided by edges of end parts, with respect to a front-back direction of the diaper, of the front part and the back part. Here, the front-back direction means a direction from the front part toward the back part of the pants-type disposable diaper. The width direction means a direction orthogonal to the front-back direction on the same plane as the pants-type disposable diaper in a state where the front part and the back part of the pants-type disposable diaper are disjoined and the pants-type disposable diaper is developed on a plane.

Concerning names of respective parts of the pants-shaped outer member, a part applied to an abdomen side of a wearer is called the front part, a part applied to a buttocks side of the wearer is called the back part, and a part positioned between the front part and the back part and applied to a crotch of the wearer is called the crotch part, in a state of wearing the pants-type disposable diaper. The crotch part is a middle part when the pants-type disposable diaper is divided into three parts in the front-back direction in a state where the front part and the back part of the diaper are disjoined and the diaper is developed on a plane, and the crotch part is a part whose side edges in the width direction are not joined when the diaper is formed in a shape of pants.

The absorbent main body is disposed on the inner surface of the pants-shaped outer member at the crotch part. The inner surface means a surface that faces a wearer's skin in wearing the pants-type disposable diaper. An outer surface means a surface that faces outside in wearing the pants-type disposable diaper. The absorbent main body is disposed at least at the crotch part, and further, may be also extended to the front part and/or the back part. The absorbent main body absorbs urine and the like excreted from a wearer.

The pants-shaped outer member may be composed of one sheet that is formed into a predetermined shape, or may be composed of a laminate of two or more sheets that is formed into a predetermined shape. The pants-shaped outer member comprises a later-described first sheet, second sheet and an elastic member.

The first sheet is preferably made of a liquid-impermeable or water-repellent nonwoven fabric in light of leakage prevention. Thus, a nonwoven fabric that is formed from a hydrophobic fiber is preferable as the nonwoven fabric. In light of degree of hydrophobicity and ease of production, a polyolefin and/or polyester fiber is preferable as the hydrophobic fiber. However, a polyolefin and/or polyester nonwoven fabric is inferior softness, and may be felt stiff when handled. For that reason, in the present invention, when a nonwoven fabric used for the first sheet is produced, a nonionic surfactant is used in addition to polyolefin and/or polyester as a raw material of the nonwoven fabric. Thus, a nonwoven fabric produced by melting a polymer composition containing polyolefin and/or polyester and a nonionic surfactant is used as the first sheet. As a result, softness is imparted to the first sheet.

In the pants-type disposable diaper of the present invention, for example, in the case that the first sheet is disposed so as to be exposed to outside, a hand feel of the diaper improves when a caregiver or the like handles the diaper, thereby providing a comfortable feeling. In the case that the first sheet is disposed so as to face a wearer, a skin feel of the diaper against the wearer is improved.

In the present invention, a nonionic surfactant is used as one component of the polymer composition, and the nonwoven fabric is produced by melting the polymer composition, whereby softness is imparted to the nonwoven fabric without impairing hydrophobicity of the polyolefin and/or polyester nonwoven fabric at a marked degree. For example, when a polyolefin and/or polyester nonwoven fabric is used as a nonwoven fabric and the nonwoven fabric is immersed in a surfactant or a surfactant is applied to a surface of the nonwoven fabric, softness can be imparted to the nonwoven fabric but the polyolefin and/or polyester nonwoven fabric is hydrophilized. Thus, it is difficult to obtain a liquid-impermeable or water-repellent nonwoven fabric. However, in the present invention, since a nonionic surfactant is blended in a raw material of a fiber for forming a nonwoven fabric to obtain the polymer composition, hydrophobicity provided by the polyolefin and/or polyester is maintained in the obtained nonwoven fabric, and hence the nonwoven fabric becomes excellent in water resistance. Therefore, a liquid-impermeable or water-repellent nonwoven fabric with softness is obtained.

As the polyolefin contained in the polymer composition, a polyolefin that is commonly used for a nonwoven fabric can be used. For example, polyethylene, polypropylene, polybutene-1, polyisobutylene or the like may be used. Among them, polyethylene and/or polypropylene are preferable in light of ease and cost of production. These polyolefins may be used either alone or as a combination of at least two of them.

As the polyester contained in the polymer composition, a polyester that is commonly used for a nonwoven fabric can be used. For example, polyethylene terephthalate (PET), polyeuylene terephthalate (PBT), polytrimethylene terephthalate (PTT) or the like may be used. These polyesters may be used either one or as a combination of at least two of them.

As the surfactant, a nonionic surfactant is used. Since a nonionic surfactant has a hydrophilic part of nonionic, using a nonionic surfactant as one component of the polymer composition enables easily to maintain hydrophobicity of the polyolefm and/or polyester nonwoven fabric. In addition, a nonionic surfactant has a relatively excellent affinity with polyolefin and polyester, and hence softness is easily imparted to the nonwoven fabric.

Examples of the nonionic surfactants include polyalcohol fatty acid esters such as sucrose fatty acid esters, glycerin fatty acid esters, and sorbitan fatty acid esters; polyoxyethylene alkyl ethers; polyoxyethylene alkyl phenyl ether; polyoxyethylene fatty acid esters; and polyoxyethylene polyalcohol fatty acid esters. These nonionic surfactants may be used either alone or as a combination of at least two of them.

A content of the surfactant in the polymer composition is preferably 0.5 mass % or more, more preferably 1.0 mass % or more, preferably 5.0 mass % or less, and more preferably 3.5 mass % or less. When the content of the surfactant in the polymer composition is in the range of from 0.5 mass% to 5.0 mass %, softness and hydrophobicity of the nonwoven fabric which is prepared by melting the polymer composition are ensured sufficiently.

As the nonwoven fabric constituting the first sheet, a spunbonded nonwoven fabric is preferable. Here, the spunbonded nonwoven fabric means a nonwoven fabric 20 manufactured by a spunbonding method. The spunbonded nonwoven fabric is obtained, for example, by: melting the polymer composition; extruding the polymer composition from a spinning nozzle to be extended; collecting the extended polymer composition on a conveyor belt or the like to be formed into a web shape. In the present invention, since the spunbonded nonwoven fabric is used as the first sheet, breathability is ensured and imperviousness of moisture during wearing the diaper is easily prevented.

In the present invention, the spunbonded nonwoven fabric means a nonwoven fabric consisting of a spunbond layer. For example, an SMS nonwoven fabric in which a meltblown layer is interposed between spunbond layers is not preferable, because the meltblown layer impairs breathability. In addition, as described above, in the present invention, since the first sheet has a sufficient water resistance, it is unnecessary to provide a meltblown layer for enhancing water resistance of the first sheet.

The nonwoven fabric used for the first sheet has preferably a mass per unit area of 10 g/m² or more, more preferably 15 g/m² or more, preferably 35 g/m² or less, and more preferably 25 g/m² or less. When the nonwoven fabric has a mass per unit area of 10 g/m² or more, the first sheet tends to have a sufficient strength. When the nonwoven fabric has a mass per unit area of 35 g/m² or less, breathability of the nonwoven fabric is easily ensured, resulting in improving a comfort of a wearer.

The nonwoven fabric used for the first sheet has preferably a fineness of 1.0 dtex or more and less than 1.5 dtex. When the nonwoven fabric has a fineness of less than 1.5 dtex, softness is easily imparted to the nonwoven fabric. When the nonwoven fabric has a fineness of 1.0 dtex or more, the spunbonded nonwoven fabric is easily produced.

As described above, in the pants-type disposable diaper of the present invention, since the pants-shaped outer member comprises the first sheet made of the spunbonded nonwoven fabric produced by melting the polymer composition containing polyolefin and/or polyester and the nonionic surfactant, the pants-shaped outer member is excellent in water resistance and breathability, and further excellent in softness.

The pants-shaped outer member comprises a second sheet laminated on an inner surface or an outer surface of the first sheet and not containing a surfactant. As described above, since the first sheet is made of the nonwoven fabric containing the nonionic surfactant, the first sheet is excellent in softness. However, when the pants-shaped outer member has only one or more of the first sheet as a sheet member, the pants-shaped outer member may become excessively soft, thereby deteriorating handleability during manufacturing or wearing of the diaper. For that reason, in the present invention, in light of imparting rigidity to the pants-shaped outer member to improve handleability of the diaper, the pants-shaped outer member preferably has the second sheet in addition to the first sheet.

The second sheet may be made of a hydrophilic nonwoven fabric or made of a liquid-impermeable or water-repellent nonwoven fabric; however the second sheet is preferably made of a nonwoven fabric having breathability.

A material of the nonwoven fabric used for the second sheet can be selected as appropriate from synthetic fibers such as polypropylene, polyethylene, polyester and polyamide; natural fibers such as pulp and silk. In addition, a composite fiber having a core-in-sheath structure or a side-by-side structure may be used. The above-mentioned fibers may be used either alone or as a combination of at least two of them.

As the nonwoven fabric constituting the second sheet, a nonwoven fabric prepared from a hydrophobic material such as polypropylene, polyethylene and polyester is preferable. When such a nonwoven fabric is used, the second sheet with high strength is easily obtained. In addition, because the second sheet does not contain a surfactant, using a nonwoven fabric prepared from a hydrophobic material makes the second sheet liquid-impermeable or water-repellent, thereby preventing leakage of urine and the like from the pants-shaped outer member.

A manufacturing method of the nonwoven fabric constituting the second sheet is not particularly limited; however, the nonwoven fabric constituting the second sheet is preferably a nonwoven fabric manufactured by a spunbonding method, an air-through method, a point bonding method, a spunbonding-meltblowing-spunbonding method (an SMS method), a chemical bonding method, or the like, in light of ensuring breathability.

The nonwoven fabric used for the second sheet has preferably a mass per unit area of 10 g/m² or more, more preferably 15 g/m² or more, preferably 35 g/m² or less, and more preferably 25 g/m² or less. When the nonwoven fabric has a mass per unit area of 10 g/m² or more, the second sheet tends to have a sufficient strength. When the nonwoven fabric has a mass per unit area of 35 g/m² or less, breathability of the nonwoven fabric is easily ensured, resulting in improving a comfort of a wearer.

In the pants-shaped outer member, the second sheet, that does not contain a surfactant, is preferably laminated on the inner surface of the first sheet. The absorbent main body is disposed on the inner surface of the pants-shaped outer member, and when the absorbent main body is fixed to the second sheet having a certain degree of rigidity, the absorbent main body is easily set at a desired position in a crotch of a wearer in wearing the diaper, thereby improving handleability of the diaper.

In the case that the second sheet is laminated on the inner surface of the first sheet, the first sheet is preferably folded back toward the second sheet at a waist opening edge of the pants-shaped outer member. Here, the waist opening edge means an edge of the waist opening in the pants-shaped outer member. In the pants-type disposable diaper of the present invention, since the first sheet has softness, when the first sheet is folded back toward the second sheet at the waist opening edge of the pants-shaped outer member, a wearer's skin contacts to the first sheet at around the waist opening of the pants-shaped outer member and a skin feel of the pants-shaped outer member is improved.

The pants-shaped outer member comprises the second sheet laminated on the first sheet and an elastic member is disposed between the first sheet and the second sheet. The elastic member may be adhered to the first sheet and/or the second sheet.

A waist elastic member is preferably disposed to the pants-shaped outer member along the waist opening edge at the front part and the back part. The waist elastic member prevents excrement such as urine and the like from leaking from a back side or an abdomen side, even when a wearer lies. The waist elastic member may be composed of a plurality of elastic members.

A leg elastic member is preferably disposed to the pants-shaped outer member along a leg opening edge. The leg elastic member prevents excrement such as urine and the like from leaking from the leg opening edge. Here, the leg opening edge means an edge of the leg opening in the pants-shaped outer member. The leg elastic member may be composed of a plurality of elastic members.

A plurality of body elastic members are preferably disposed to the pants-shaped outer member at the front part and the back part so as to extend in the width direction of the diaper. The body elastic member improves a fitting property of the diaper around abdomen and hip regions.

When the body elastic member is disposed to the pants-shaped outer member, the first sheet and the second sheet are not adhered each other at a position between the adjacent body elastic members, preferably. When the first sheet and the second sheet are not adhered each other at the position between the adjacent body elastic members, a hand feel of the diaper is further improved around a wearer's trunk.

Elastic materials such as a polyurethane thread, a polyurethane film, a natural rubber and the like, which are generally used for disposable diapers, can be used for the respective elastic members. The respective elastic members are preferably fixed to the first sheet and/or the second sheet in a stretched state with a hot-melt adhesive. For example, a polyurethane thread having a fineness of 100 dtex to 2,500 dtex is stretched at a ratio of 1.1 to 5.0 times to be fixed. A preferable bonding means is a rubber hot-melt adhesive.

In order not to adhere the first sheet to the second sheet at the position between the adjacent body elastic members, the following method may be employer. As an adhesive applicator; an adhesive application nozzle in which a pair of guides extends from a nozzle discharge opening is used, and the adhesive application nozzles, the number of which is equal to the number of elastic members, are arranged. While the adhesive is discharged from the nozzle discharge opening, each elastic member is reeled out through the paired guides to apply the adhesive around the elastic member. The plurality of elastic members to which the adhesive has been applied are disposed on the first sheet Then, the second sheet is laminated on the first sheet to which the elastic members have been fixed, and the first sheet and the second sheet are joined to each other by means of an adhesive, heat-sealing, or the like. It is noted that the above-described adhesive application nozzle is disclosed in Japanese Laid-Open Patent Publication No. 2009-11890.

The absorbent main body disposed on the inner surface of the pants-shaped outer member at the crotch part comprises a top sheet, a back sheet, and an absorbent core disposed between the top sheet and the back sheet. The top sheet is preferably made of a liquid-permeable nonwoven fabric or the like, and the back sheet is preferably made of a nonwoven fabric, a plastic film or the like which is liquid-impermeable or water-repellent.

The absorbent core is not particularly limited as long as it absorbs excrement such as urine and the like; however, it preferably contains an absorbent resin. The absorbent core can be obtained, for example, by the steps of mixing a hydrophilic fiber assembly such as crushed pulp fibers, cellulose fibers and the like with a granular absorbent resin to obtain a clump; wrapping the clump with a paper sheet such as a tissue paper and the like, or with a cover sheet such as a liquid-permeable nonwoven fabric sheet and the like; and molding the obtained wrapped clump into a predefined shape such as a rectangular shape, an hourglass shape, a center nipped-in gourd shape, a battledore shape, and the like.

Rising flaps are preferably provided along edges of opposite sides, with respect to the width direction, of the absorbent main body. For example, the rising flaps may be joined to the top sheet of the absorbent main body, or may be provided outside the absorbent main body in the width direction. The rising flaps are preferably made of a nonwoven fabric, a plastic film or the like which is liquid-impermeable or water-repellent, and more preferably made of a water-repellent nonwoven fabric. Providing the rising flaps enables to prevent lateral leakage of urine and the like.

A rising elastic member is preferably disposed at an upper end (an end nearer to a wearer) of the rising flap being in a rising state. The rising flap forms a rising gather which rises toward a wearer due to a shrinkage force of the rising elastic member, thereby preventing lateral leakage of urine and the like. An inner surface of the rising flap may be joined to the top sheet at ends, with respect to the front-back direction of the diaper, of the rising flaps, thereby preventing leakage of urine and the like in the front-back direction.

Next, an example of the pants-type disposable diaper of the present invention is explained, referring to drawings. However, the present invention is not restricted to the following embodiment.

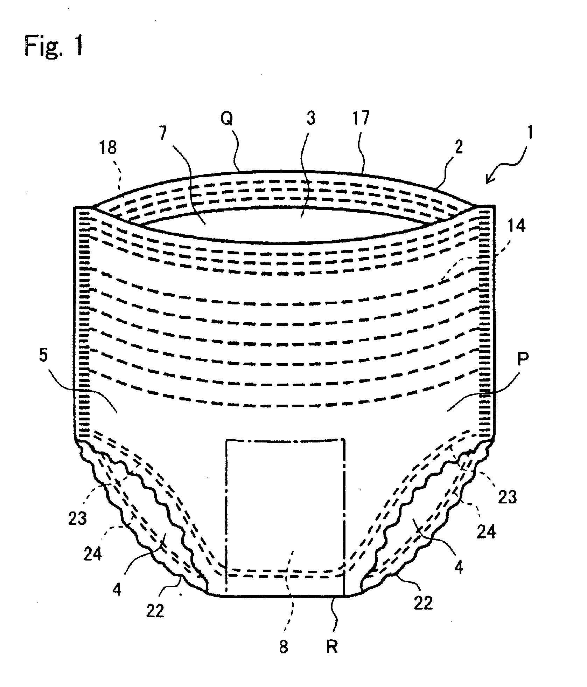

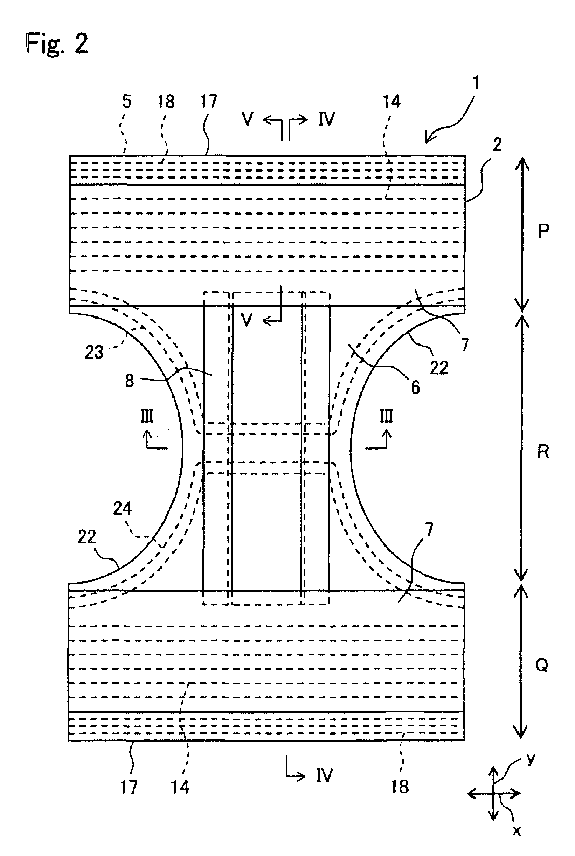

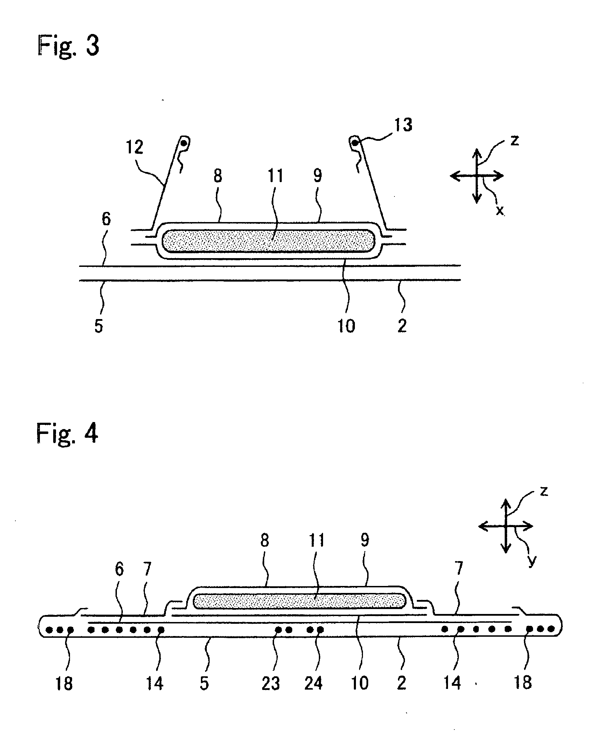

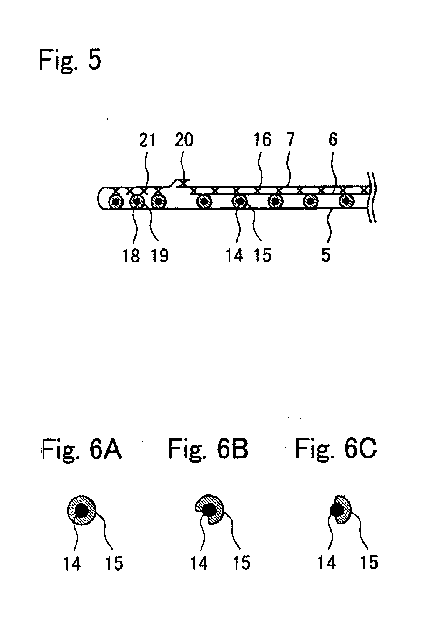

Fig. 1 shows a perspective view of a pants-type disposable diaper of the present invention. Fig. 2 shows a plan view of the pants-type disposable diaper shown in Fig. 1 in a developed state in which a front part and a back part are disjoined. Fig. 3 shows a cross-sectional view taken along line III-III in Fig. 2. Fig. 4 shows a cross-sectional view taken along line IV-IV in Fig. 2. Fig. 5 shows a cross-sectional view taken along line V -V in Fig. 2. In the drawings, the arrow x direction is defined as a width direction of the diaper, and the arrow y is defined as a front-back direction of the diaper. The direction perpendicular to the plane formed by the arrows x and y is defined as a thickness direction z.

A pants-type disposable diaper 1 comprises a pants-shaped outer member 2 having, a front part P, a back part Q, and a crotch part R positioned between the front part P and the back part Q, and having a waist opening 3 and a pair of leg openings 4 formed by joining the front part P and the back part Q. The pants-shaped outer member 2 comprises: a first sheet 5 made of a spunbonded nonwoven fabric produced by melting a polymer composition containing polyolefin and/or polyester and a nonionic surfactant; and a second sheet 6 laminated on an inner surface of the first sheet 5 and not containing a surfactant.

The pants-type disposable diaper 1 comprises an absorbent main body 8 disposed on an inner surface of the pants-shaped outer member 2 at the crotch part R and comprising a top sheet 9, a back sheet 10, and an absorbent core 11 disposed between the top sheet 9 and the back sheet 10 (Fig. 3). The top sheet 9 is placed so as to face a wearer's skin, and allows excrement such as urine and the like to permeate through. The excrement that permeated the top sheet 9 is accommodated in the absorbent core 11. The back sheet 10 is attached to the second sheet 6 of the pants-shaped outer member 2, and prevents the excrement from leaking outside.

Rising flaps 12 are provided along an edge of opposite sides, with respect to the width direction x, of the absorbent main body 8. The rising flap 12, which extends in the front-back direction y of the diaper, is joined astride the top sheet 9 and the back sheet 10. A rising elastic member 13 is disposed at an inner end in the width direction of the rising flap 12. A rising gather which rises upward (toward a wearer) is formed from the rising flap 12 due to a shrinkage force of the rising elastic member 13, thereby preventing lateral leakage of urine and the like. An inner surface of the rising flaps 12 is joined to the top sheet 9 at front and back ends of the absorbent main body 8, thereby preventing leakage of urine and the like outward in the front-back direction y.

A plurality of body elastic members 14 are disposed between the first sheet 5 and the second sheet 6 at the front part P and the back part Q of the pants-shaped outer member 2 so as to extend in the width direction x of the diaper (Figs. 1,2 and 4). The body elastic member 14 is adhered to the first sheet 5 and the second sheet 6 with a hot-melt adhesive 15 applied around the body elastic member 14. The first sheet 5 and the second sheet 6 are not adhered each other at a position between the adjacent body elastic members 14, as shown in Fig. 5.

Concerning application of the hot-melt adhesive 15 around the body elastic member 14, the hot-mett adhesive 15 may be applied to the entire circumference of the body elastic member 14 as shown in Fig. 6A, or the hot-melt adhesive 15 may be applied to substantially 3/4 or half of the circumference of the body elastic member 14 as shown in Figs. 6B or 6C. In light of reliable adhesion of the body elastic member 14 to the first sheet 5 and the second sheet 6, the hot-melt adhesive 15 is preferably applied to at least half or more of the circumference of the body elastic member 14. Application of the hot-melt adhesive to the entirety or a part of the circumference of the elastic member as shown in Figs. 6A to 6C is not limited to application to the body elastic member 14.

In Figs. 2 and 4, an end-holding sheet 7 is provided at the front part P and the back part Q of the pants-type disposable diaper 1 so as to cover a front or back end of the absorbent main body 8. The end-holding sheet 7 is provided so that a wearer is less likely to feel discomfort that is caused by the front or back end of the absorbent main body 8 coming into direct contact with a wearer's skin. In Figs. 4 and 5, the end-holding sheet 7 is adhered to the front or back end of the absorbent main body 8 and the second sheet 6 with a hot-melt adhesive 16 so as to cover the front or back end of the absorbent main body 8 and the entirety of the body elastic members 14.

A plurality of waist elastic members 18 are disposed to the pants-shaped outer member 2 so as to extend along a waist opening edge 17, that is an edge of the waist opening 3, in the width direction x of the diaper, as shown in Figs. 1, 2 and 4. The first sheet 5 is folded back toward the second sheet 6 at the waist opening edge 17 of the pants-shaped outer member 2, and the folded first sheet 5 is adhered to the end-holding sheet 7 with a hot-melt adhesive 20, as shown in Fig. 5. The waist elastic members 18 are interposed between the folded and unfolded parts of the first sheet 5, and adhered to the first sheet 5 with the hot-melt adhesive 19 applied around the waist elastic member 18. In order to reliably join the waist elastic members 18 to the first sheet 5, preferably, a hot-melt adhesive 21 is applied to the folded part of the first sheet 5 and the waist elastic members 18 are adhered to the first sheet 5 with the hot-melt adhesive 21.

Leg elastic members 23 and 24 are disposed between the first sheet 5 and the second sheet 6 along the leg opening edges 22, that is edges of the leg openings 4, of the pants-shaped outer member 2 , as shown in Fig. 2. The leg elastic member consists of: a front leg elastic member 23 provided so as to extend across the crotch part R and along the leg opening edges on a front side of the diaper; and a back leg elastic member 24 provided so as to extend across the crotch part R and along the leg opening edges on a back side of the diaper. By the front leg elastic member 23 and the back leg elastic member 24, the leg elastic member is provided along substantially the entire circumference of the leg opening edges 22.

### REFERENCE SIGNS LIST

1: a pants-type disposable diaper
2: a pants-shaped outer member
5: a first sheet
6: a second sheet
8: an absorbent main body
9: a top sheet
10: a back sheet
11: an absorbent core
14: a body elastic member
15: a hot-melt adhesive
18: a waist elastic member
23, 24: a leg elastic member

## Claims

1. A pants-type disposable diaper (1) comprising:
a pants-shaped outer member (2) having a front part (P), a back part (Q), and a crotch part (R) positioned between the front part (P) and the back part (Q), and having a waist opening (3) and a pair of leg openings (4) formed by joining the front part (P) and the back part (Q); and
an absorbent main body (8) disposed on an inner surface of the pants-shaped outer member (2) at the crotch part (R), and comprising a top sheet (9), a back sheet (19), and an absorbent core (11) disposed between the top sheet (9) and the back sheet (10); wherein
the pants-shaped outer member (2) comprises:
a first sheet (5) made of spunbonded nonwoven fabric produced by melting a polymer composition containing polyolefin and/or polyester and a nonionic surfactant;
a second sheet (6) laminated on an inner surface or an outer surface of the first sheet (5), made of nonwoven fabric, and not containing a surfactant; and
an elastic member (14, 18, 23, 24) disposed between the first sheet (5) and the second sheet (6).

2. The pants-type disposable diaper (1) according to claim 1, wherein the second sheet (6) is laminated on the inner surface of the first sheet (5), and the first sheet (5) is folded back toward the second sheet (6) at an edge (17) of the waist opening (3) of the pants-shaped outer member (2).

3. The pants-type disposable diaper (1) according to claim 1 or 2, wherein
a plurality of body elastic members (14) are disposed between the first sheet (5) and the second sheet (6) at the front part (P) and the back part (Q) of the pants-shaped outer member (2) so as to extend in a width direction of the diaper as the elastic member, the body elastic members (14) are adhered to the first sheet (5) and/or the second sheet (6), and
the first sheet (5) and the second sheet (6) are not adhered each other at a position between the adjacent body elastic members (14).

4. The pants-type disposable diaper (1) according to any one of claims 1 to 3, wherein the nonwoven fabric of the first sheet (5) has a fineness of 1.0 dtex or more and less than 1.5 dtex.

## Patentansprüche

1. Höschenartige Wegwerfwindel (1), Folgendes umfassend:
ein höschenförmiges äußeres Element (2) mit einem vorderen Teil (P), einem hinteren Teil (Q) und einem Schrittteil (R), der zwischen dem vorderen Teil (P) und dem hinteren Teil (Q) anbeordnet ist, und mit einer Taillenöffnung (3) und einem Paar Beinöffnungen (4), die durch das Verbinden des vorderen Teils (P) und des hinteren Teils (Q) gebildet sind; und
einen absorbierenden Hauptkörper (8), der an einer Innenfläche des höschenförmigen äußeren Elements (2) am Schrittteil (R) angeordnet ist und eine obere Lage (9), eine untere Lage (19) und einen absorbierenden Kern (11) umfaßt, der zwischen der oberen Lage (9) und der unteren Lage (10) angeordnet ist; wobei
das höschenförmige äußere Element (2) Folgendes umfasst:
eine erste Lage (5), die aus Spinnvlies hergestellt ist, das durch Schmelzen einer Polymerzusammensetzung erzeugt wird, die Polyolefin und/oder Polyester und ein nicht ionisches Tensid enthält;
eine zweite Lage (6), die an eine Innenfläche oder eine Außenfläche der ersten Lage (5) laminiert und aus Vlies hergestellt ist und kein Tensid enthält; und
ein elastisches Element (14, 18, 23, 24), das zwischen der ersten Lage (5) und der zweiten Lage (6) anbeordnet ist.

2. Höschenartige Wegwerfwindel (1) nach Anspruch 1, wobei die zweite Lage (6) an die Innenfläche der ersten Lage (5) laminiert ist und die erste Lage (5) an einem Rand (17) der Taillenöffnung (3) des höschenförmigen äußeren Elements (2) zur zweiten Lage (6) zurückgefaltet ist.

3. Höschenartige Wegwerfwindel (1) nach Anspruch 1 oder 2, wobei
zwischen der ersten Lage (5) und der zweiten Lage (6) am vorderen Teil (P) und am hinteren Teil (Q) des höschenförmigen äußeren Elements (2) mehrere körperelastische Elemente (14) derart angeordnet sind, dass sie sich in einer Breiterichtung der Windel als elastisches Element erstrecken, die körperelastischen Elemente (14) an der ersten Lage (5) und/oder an der zweiten Lage (6) haften, und
die erste Lage (5) und/oder die zweite Lage (6) an einer Position zwischen den nebeneinander legenden körperelastischen Elementen (14) nicht aneinander haften.

4. Höschenartige Wegwerfwindel (1) nach einem der Ansprüche 1 bis 3, wobei das Vlies der ersten Lage (5) einen Feinheitsgrad von 1,0 dtex oder mehr und weniger als 1,5 dtex aufweist.

## Revendications

1. Couche-culotte jetable (1) comprenant :
un élément extérieur de type couche-culotte (2) comportant une partie avant (P), une partie arrière (Q) et une partie entrejambe (R) positionnée entre la partie avant (P) et la partie carrière (Q) et ayant une couverture de taille (3) et une paire d'ouvertures de jambes (4) formées en joignant la partie avant (P) et la partie arrière (Q) ; et
un corps absorbant principal (8) disposé sur une surface interne de l'élément extérieur de type couche-culotte (2) au niveau de la partie entrejambe (R) et comprenant une feuille supérieure (9), une feuille arrière (19) et un noyau absorbant (11) disposé entre la feuille supérieure (9) et la feuille arrière (10) ;
l'élément extérieur de type couche-culotte (2) comprenant :
une première feuille (5) en non-tissé lié par filage produit en faisant fondre une composition de polymère contenant de la polyoléfine et/ou du polyester et un surfactant non ionique ;
une seconde feuille (6) laminée sur une surface interne ou une surface externe de la première feuille (5) en non-tissé et ne contenant pas de surfactant ; et
un élément élastique (14, 18, 23, 24) disposé entre la première feuille (5) et la seconde feuille (6).

2. Couche-culotte jetable (1) selon la revendication 1, dans laquelle la seconde feuille (6) est laminée sur la surface interne de la première feuille (5) et la première feuille (5) est repliée vers la seconde feuille (6) au niveau d'un bord (17) de l'ouverture de traille (3) de l'élément extérieur de type couche-culotte (2).

3. Couche-culotte étable (1) salon la revendication 1 ou 2, dans laquelle
une pluralité d'éléments à corps élastiques (14) sont disposés entre la première feuille (5) et la seconde feuille (6) au niveau de la partie avant (P) et de la partie carrière (Q) de l'élément extérieur de type couche-culotte (2) de manière à s'étendre dans le sens de la largueur de la couche lorsque l'élément élastique, les éléments à corps élastiques (14) adhèrent sur la première feuille (5) et/ou la seconde feuille (6), et
la première feuille (5) et la seconde feuille (6) n'adhèrent pas ensemble à une position située entre les éléments à corps élastiques (14) adjacents.

4. Couche-culotte étable (1) selon l'une quelconque des revendications 1 à 3, dans laquelle le non-tissé de la première feuille (5) a une épaisseur de 1,0 dtex ou de plus ou moins 1, 5 dtex.
